(19) Europäisches Patentamt
European Patent Office
Office européen des brevets

(11) Publication number: **0 158 170 B1**

(12) # EUROPEAN PATENT SPECIFICATION

(45) Date of publication of patent specification: **04.01.89**

(51) Int. Cl.⁴: **C 07 H 17/08,** C 07 C 69/40, A 61 K 31/70, A 61 K 31/225

(21) Application number: **85103177.3**

(22) Date of filing: **19.03.85**

(54) **Erythromycin A salt with mucosecretolytic and fluidizing activity, a process for its preparation and pharmaceutical compositions thereof.**

(30) Priority: **02.04.84 IT 2035484**

(43) Date of publication of application: **16.10.85 Bulletin 85/42**

(45) Publication of the grant of the patent: **04.01.89 Bulletin 89/01**

(84) Designated Contracting States: **AT BE CH DE FR GB IT LI LU NL SE**

(56) References cited:
**EP-A-0 096 116**
**CHEMICAL ABSTRACTS, vol. 90, no. 10, 5th March 1979, page 8, no. 80589v, Columbus, Ohio, US; K. TSUJI et al.: "Fluorimetric determination of erythromycin and erythromycin ethylsuccinate in serum by a high-performance liquid chromatographic post-column, on-stream derivatization and extraction method", & J. CHROMATOGR. 1978, 158, 337-43**

**The file contains technical information submitted after the application was filed and not included in this specification**

(73) Proprietor: **CAMILLO CORVI S.p.A.**
**via S. Marco 18**
**Milan (IT)**

(72) Inventor: **Corvi Mora, Camillo**
**viale dei Mille 3**
**Piacenza (IT)**

(74) Representative: **Klausner, Erich et al**
**c/o Ufficio Internazionale Brevetti Ing. C. Gregorj**
**S.p.A. Via Dogana 1**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 158 170 B1

Courier Press, Leamington Spa, England.

## Description

An object of this invention is the erythromycin A salt of butanedioic acid, mono[5-(1-hydroxy-1-methylethyl)-2-methyl-2-cyclohexen-1-yl]ester of the formula

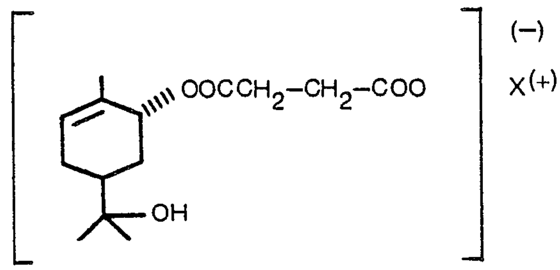

(I)

Code: CO/1311)

where $X^{(+)}$ represents the monovalent cation of erythromycin A and the anion is derived from butanedioic acid, mono[5-(1-hydroxy-1-methylethyl)-2-methyl-2-cyclohexen-1-yl]ester having the formula

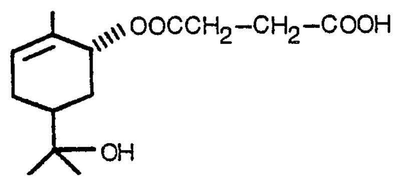

(II)

Code: CO/1032

$C_{14}H_{22}O_5$

mol. wt. 270. 32

The compound of formula II, an ester-acid, which is derived from d,l-trans-sobrerol, is novel and, therefore, falls within the present invention.

The d,l-trans-sobrerol is known, however, because of its employment in the therapy of respiratory diseases, since it acts as a mucoregulator and expectorant.

It has been found, unexpectedly, that the erythromycin A salt (I), beside having a mucosecretolytic and fluidizing activity greater than that of sobrerol, provides a novel well-balanced antibiotic complex useful for the selective therapy of the respiratory tract.

Erythromycin, as is known, is an antibiotic substance suitable for treating gram-positive infections and it is particularly effective against staphylococci, streptococci, pneumococci, which are resistant against the other antibiotic drugs. Erythromycin is further effective for treating infections caused by a mixture of gram-positive and gram-negative bacteria. The formula of erythromycin A is given herebelow:

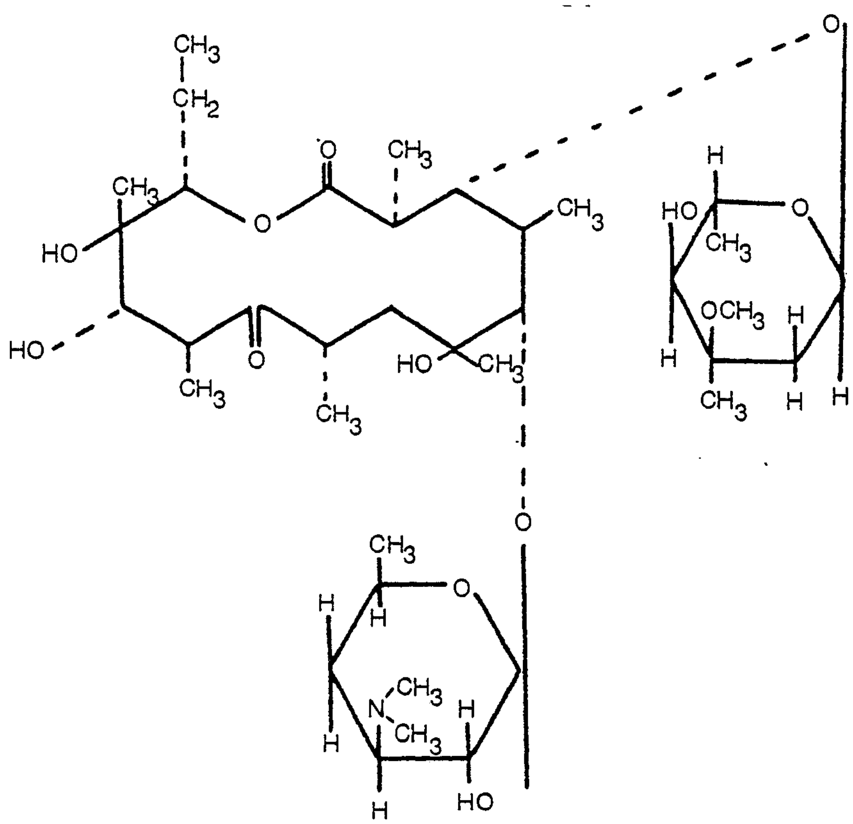

(III)

An object of the invention is, further, to provide a process for preparing the ester-acid (II) (code CO/1032) by reacting succinic anhydride (IV) with d,l-trans-sobrerol(V) according to the following scheme:

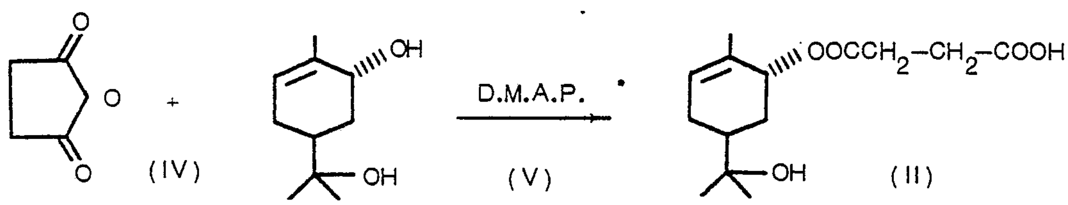


(IV) (V) (II)

$C_4H_4O_3$ mol. wt. 100.07 — $C_{10}H_{18}O_2$ mol. wt. 170.25 — $C_{14}H_{22}O_5$ mol. wt. 270.32

The reaction is carried out, in an optimum way, in an aprotic solvent such as, for example, tetrahydrofuran, dioxane, anhydrous methylene chloride free from ethanol, by catalyzing with dimethylamino-pyridine (D.M.A.P.). Temperature is kept within the range of from 20 to 25°C over a time of 8 to 20 hours, preferably for about 12 hours.

Then, the temperature is raised to reflux (65—66°C) at which it is kept for 2 to 5 hours, preferably 3 hours. The so obtained crystalline acid product is redissolved, by adding the same to an equivalent solution of erythromycin base in methylene chloride. The salt (I) which forms is precipitated with isopropyl ether after concentration.

The process according to this invention is illustrated in the following non limitative examples.

Example

Butanedioic acid, mono[5-(1-hydroxy-1-methylethyl)-2-methyl-2-cyclohexen-1-yl]ester (II) (see the reaction scheme above).

A mixture of 17 g (0.1 mole) d,l-trans-sobrerol, 200 ml tetrahydrofuran, 11 g (0.11 mole) succinic anhydride and 3 g (0.025 mole) 4-dimethylaminopyridine (mol. wt. 122.17) as the catalyst, is stirred 12 hours at room temperature and then refluxed (66°C) for 3 hours. The reaction mixture is then poured into 400 of 5% sulphuric acid aqueous solution, and stirred for 1 hour. It is extracted with ethyl acetate (3 × 300 ml). The combined organic phases are washed with water (3 × 100 ml) and dried on anhydrous $MgSO_4$. The

solvent is concentrated, under reduced pressure, to a volume of about 100 ml and it is cooled in ice-water. It is filtered under vacuum thereby obtaining, after drying, 24.3 g of a white crystalline product, m.p. 123—125°C. Theoretic yield = 90%.

## Example 2

Erythromycin A salt of butanedioic acid, mono-[5-(1-hydroxy-1-methylethyl)-2-methyl-2-cyclohexen-1-yl]ester (I)

Erythromycin A base
$C_{37}H_{67}NO_{13}$
mol. wt. 733.92 (III) +

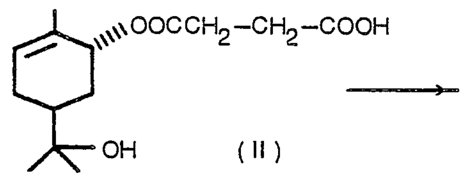

(II)

$C_{14}H_{22}O_5$
mol. wt. 270.32

Erythromycin A salt (I)
$C_{51}H_{89}O_{18}$ mol. wt. 1004.24

To a solution of 50 g (0.068 mole) erythromycin A base in 600 ml of methylene chloride 18.4 g (0.068 mole) butanedioic acid, mono[5-(1-hydroxy-1-methylethyl)-2-methyl-2-cyclohexen-1-yl]ester is added. The mixture is left under stirring ½ hour (clear solution). 1 litre of isopropyl ether is added and the solution is concentrated to a volume of about 800 ml. The solution is cooled in ice-water and is filtered under reduced pressure, yielding the white, crystalline erythromycin A salt. Yield = g.65 (95% of theoretical), m.p. = 80—100°C.

Analytical characterization of the compound of formula (II)

1. Elemental analysis for $C_{14}H_{22}O_5$, mol. wt. 270.328
   Calculated, % C=62.21 H=8.2 O=29.59
   Found, % C=62.44 H=8.14
(Average of 3 tests)

2. I.R. spectrum (nujol dispersion; $cm^{-1}$)
   3415 γ OH
   2320—2880 γ OH acid (several little bands)
   1725
   C=O ester and acid
   1710
   1320—1208; 1155 and 918 (characteristic bands)

3. $H^1$ N.M.R. spectrum ($CDCl_3$ solvent; internal reference T.M.S. δ p.p.m.)
   5.67 centre c.a. (1H = *CH*)
   5.22 centre, c.a. (1H; $W_{\frac{1}{2}}$ = 8Hz; *CH*OCO)
   2.62 b.s. (4H; OC—$CH_2$—$CH_2$—CO)
   2.5 + 1.3 c.a. (5H; = C—$CH_2$—*CH*—$CH_2$—CO)
   1.68 b.s. (3H; = C—$CH_3$)
   1.2 and 1.12 s. (3H each, gem. $CH_3$)
   Legend: c.a. = complex absorption
   b.s. = broadened singlet
   s. = singlet
   T.M.S. = tetramethylsilane
   $W_{\frac{1}{2}}$ = broadness at half height.

4. Mass spectrum (quadrupole, direct insertion, 80 eV, 70 mA; m/z)
252 $(M—18)^+$; 0.56%) 170 (5%); 152 (42%); 137 (19); 134 (10); 119 (59); 109 (89); 94 (68); 93 (46); 79 (0.9); 59 (base peak).

Characterization of the compound of formula (I)

1. Elemental analysis for $C_{51}H_{89}NO_{18}$, mol.wt. 1004.273
   Calculated, % C=61.00 H=8.93 N=1.39 O=28.68
   Found, % C=61.19 H=8.86 N=1.31
(Average of 3 tests)

2. I.R. spectrum (nujol dispersion, $cm^{-1}$)
3470 γ OH (broadened)
1723 γ CO (ester, lactone, ketone group)
1585 γ as $COO^-$
1165 and 1012 characteristic bands

3. $H^1$ N.M.R. spectrum ($CDCl_3$ solvent; internal reference T.M.S.; δ p.p.m.)
6.68 centre b.s. (1H = *CH*)
2.22 centre b.s. (1H $W_{\frac{1}{2}} \cong$ 8Hz; *CH*OCO terpene fraction
3.27 s. (3H; $CH_3$—O)
2.57 and 2.53 s. (4H and 6H; O—CO—$CH_2$—$CH_2$—CO and $N^+$ $(CH_3)_2$
1.67 b.s. (3H; $CH_3$—C=)

Legend: see N.M.R. spectrum above.

Acute Toxicity

Method for studying $LD_{50}$ in the mouse after a single oral administration.

Groups of 10 albino Swiss, female, adult (g.20—22) mice, fasting from the evening preceding the test, are administered orally various doses (4 to 5) of the test drugs, dissolved/suspended in 1% hydroxyethyl cellulose (volume administered: 20 ml/kg). Thereafter, the animals are fed again (Morini MIL mice-feed).

The 50% lethal dose ($LD_{50}$) is calculated according to Litchfield, J. T. and Wilcoxon, F. (J. Pharmacol. *96,* 99—113, 1949) by the mortality data as obtained the 14th day after the drug treatment.

TABLE NO. 1
Acute toxicity in the mouse after oral administration

| Substance | $DL_{50}$ in mg/kg |
|---|---|
| CO/1311 | 4322.3 |
| d,l-trans-sobrerol | 2340 |
| erythromycin A base | < 5000 |

Bronchosecretagogue activity

Method of mucoproduction in the rabbit according to Scuri R. et al, Boll Chim. Farm. *119,* 181—7, 1980.

Male brown rabbits weighing 2.8—3.5 kg are employed. To said animals, by surgical operation under anaesthesia, a T shaped tracheal cannula is applied, as described in the bibliographic reference mentioned above.

To the cannula a polypropylene 2 ml test tube is applied for periodical collection of the bronchial secretion. The study of mucoproduction started at the fourth day after the operation, is divided into two 4 hour periods for collecting and measuring the mucus, and exactly from 8:30 to 12:30 (I) and from 12:30 to 16:30 (II).

The activity of each drug is tested by administering the same by oesophageal way (os) at the beginning of II period and by evaluating the percent increase of mucoproduction (weight of the mucus as collected) over the II period as compared to the I period.

TABLE NO. 2
Bronchosecretague activity in the rabbit after oral administration

| Substance | Activity |
|---|---|
| CO/1311 * | 140 |
| d,l-trans-sobrerol | 100 |
| erythromycin A | 0 |

The activity of the compound of formula (I), of the present invention, is based on that of d,l-trans-sobrerol, taken equal to 100.

Fluidizing "in vivo" activity

A method for studying the viscosity of bronchial mucus of a bronchitic rabbit (R. Scuri et al—Il Farmaco, Ed.Pr. *36,* 36—48, 1981).

Male rabbits weighing 2.7—3.5 kg, made bronchitic by sulphuric acid aerosol treatment according to the method of Cantarelli (G. Cantarelli et al—Il Farmaco, Ed. Pr. *34*, 393—416, 1979), are treated with the test drugs and the bronchial mucus is collected by means of a tracheal cannula according to the procedure of R. Scuri (R. Scuri et al—Boll. Chim. Farm. *119*, 181—7, 1980). Viscosity of the mucus as withdrawn is studied by using a Contraves RM16 micro viscosimeter and is recorded by a Rheomat 15T-FC apparatus.

TABLE NO. 3

Fluidizing "in vivo" activity of bronchial mucus of bronchitic rabbits. Oral drug administration

| Substance | Activity |
|---|---|
| CO/1311 * | 132 |
| d,l–trans–sobrerol | 100 |
| erythromycin A | 0 |

The activity of the compound of formula (I) of this invention is based on that of d,l-sobrerol, taken equal to 100.

Antimicrobic activity

Microbiologic "in vitro" tests.

Tests carried out in order to evaluate the values of the Minimum Inhibiting Concentrations (MIC) are performed by the method of the substrate treated with scalar drugs concentrations, by the "multi-points inoculator" technique.

As test strains, bacteria from clinical isolation and from international culture collections are tested.

In the tables, the MIC values (mcg/ml), as obtained employing the Mueller-Hinton medium, are reported.

The MIC values in the tables refer to readings after 24 and 48 hours at 37°C.

TABLE No. 4

Antimicrobic "in vitro" activity

| Test strains | | | CO/1311 | | erythromycin | |
|---|---|---|---|---|---|---|
| 1 | Staph. aureus | 14154 | 10 | 10 | 10 | 10 |
| 2 | Staph. aureus | 6538 | 10 | 10 | 10 | 10 |
| 3 | Staph. aureus | clin | — | — | — | — |
| 4 | Staph. epiderm | clin | 10 | 10 | 10 | 10 |
| 5 | Staph. epiderm | clin | −0.1 | −0.1 | −0.1 | −0.1 |
| 6 | Microc. luteus | 9341 | −0.1 | −0.1 | −0.1 | −0.1 |
| 7 | Strept. faecalis | 8043 | 0.3 | 0.3 | 0.3 | 0.3 |
| 8 | Strept. faecalis | clin | 0.4 | 0.4 | 0.3 | 0.3 |
| 9 | Bac. subtilis | 6633 | — | — | — | — |
| 10 | Pseud. aerug. | clin | +100 | | +100 | |
| 11 | Pseud. aerug. | clin | 100 | 100 | 100 | 100 |
| 12 | Pseud. aerug. | clin | +100 | | +100 | |
| 13 | Esch. coli | 413 | 25 | 25 | 25 | 25 |
| 14 | Serr. marcescens | clin | 50 | 50 | 45 | 50 |
| 15 | Proteus vulg. X19 | | 2.5 | 2.5 | 2.5 | 2.5 |
| 16 | Cand. albicans | clin | 100 | 100 | 100 | 100 |

Legend:

\+ = greater than
− = smaller than
clin = clinic isolation

The strains with 4 figures are ATCC's.

Pharmacokinetics

Two groups of Wistar rats (180—270 g) are utilized. To the first group the compound of formula (I) of this invention, orally (transoesophageal intubation) at the dose of 800 mg/kg is administered, whilst to the second, again by the oral route, 585 mg/kg of erythromycin is administered.

Before treatment the rats were fasted for about 15 hours while allowed to drink ad libitum. In both cases at 0.5 h; 1 h; 1.5 h; 2 h; 3 h; 4 h; 6 h from administration 5 rats/time are sacrificed. The blood as collected in test tubes containing sodium heparin is centrifuged and the plasma so obtained is utilized for the quantitative analysis of erythromycin A by means of the microbiologic standard method, using Staphylococcus aureus as the test organism (P. G. Welling and W. A. Craig, J. Pharm. Sci. *67,* 1057 (1978)).

The plasmatic levels in erythromycin A so measured are significantly higher after a single administration of CO/1311 than the ones which are found after an equidose erythromycin A administration. From the same rats, in similar manner, the lungs are withdrawn and, after homogenizing the tissue, the erythromycin A levels are evaluated. The erythromycin A levels appear significantly higher following administration of the compound of formula (1) of the present invention.

These tests show an improved bioavailability of the erythromycin A administered as CO/1311, compared with that of the antibiotic administered as such. Further, the higher erythromycin A levels which are found at the pulmonary level indicate a specific action of the terpenoid moiety of CO/1311 to help the erythromycin A tropism toward the pulmonary structures.

In view of the activity exploited by the compound of this invention as a mucosecretolytic-fluidizing-antibiotic agent, the present invention also provides pharmaceutical compositions which contain the compound of the invention in unit dosage form.

The pharmaceutical forms containing the above mentioned active component are preferably those suitable for oral and rectal administration, in particular: capsules, coated tablets, granules for extemporaneous suspension, little bags and suppositories.

As excipients there may be employed, for the pharmaceutical oral forms: starch, lactose, microgranular cellulose, hydroxypropyl-methylcellulose, sorbitol and more generally the diluent, bonding lubricant, aromatizing, coating, taste masking and sweetening agents.

For the suppository form there are used as excipients triglycerides of saturated fatty acids, lecithins or phospholipids of the kind most commonly used in the pharmaceutical art.

**Claims for the Contracting States: BE CH DE FR GB IT LI LU NL SE**

1. Erythromycin salt of butanedioic acid, mono[5-(1-hydroxy-1-methylethyl)-2-methyl-2-cyclohexen-1-yl]ester of formula:

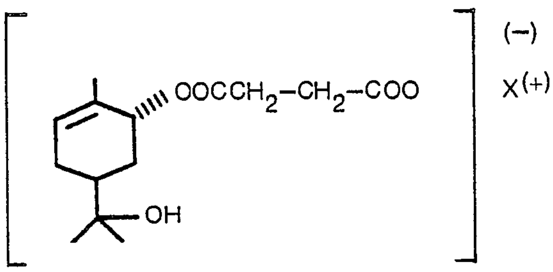

(I)

in which $X^{(+)}$ represents the monovalent cation of erythromycin A.

2. A process for the preparation of the compound of formula (I) of claim 1, characterized in that a solution of erythromycin A base (III) in an anhydrous chlorinated organic solvent is reacted, at room temperature, with an equimolar amount of butanedioic acid, mono[5-(1-hydroxy-1-methylethyl)-2-methyl-2-cyclohexen-1-yl]ester, then, to the clear solution so obtained, an ether is added in order to precipitate the erythromycin A salt of formula (I) of claim 1, whereupon the solution is concentrated to about one half the volume, cooled and filtered yielding said erythromycin A salt in crystalline form.

3. The process according to claim 2, characterized in that the reaction is carried out at a temperature from 20 to 25°C for 25—35 minutes in anhydrous methylene chloride, using isopropyl ether to precipitate the erythromycin salt.

4. Butanedioic acid, mono[5-(1-hydroxy-1-methylethyl)-2-methyl-2-cyclohexen-1-yl]ester having the formula:

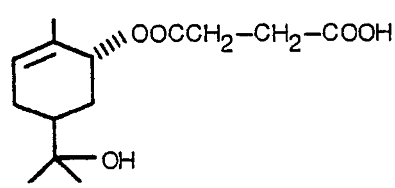

(II)

5. A process for the preparation of the acid of formula (II) of claim 4, characterized in that a mixture of d,l-trans-sobrerol in an aprotic solvent is reacted with a small excess of succinic anhydride, in the presence of 4-dimethyl-aminopyridine, stirring the reaction mixture for 8—20 hours, at room temperature, thereafter heating said reaction mixture at the reflux temperature for a 2—5 hours period, then pouring the reaction mass into a 5% sulphuric acid aqueous solution and stirring it for 50—80 minutes, extracting with ethyl acetate, washing the resulting organic phases with water and drying, the solvent is then concentrated and the reaction mass is cooled, after which it is filtered yielding the compounds of formula (II) in crystalline form.

6. The process according to claim 5, characterized in that the aprotic solvent is tetrahydrofuran and that 4-di-methylaminopyridine is added at an amount of 0.025 mole per mole of d,l-trans-sobrerol, at a temperature of 20—25°C in about 12 hours, and refluxing the reaction mass 3 hours.

7. Pharmaceutical compositions with mucosecretolytic-fluidizing and antibiotic activity, characterized in that they contain, as an active constituent, the compound of formula (I) of claim 1, together with one or more pharmacologically acceptable vehicles.

**Claims for the Contracting State: AT**

1. A process for the preparation of the compound of formula (I)

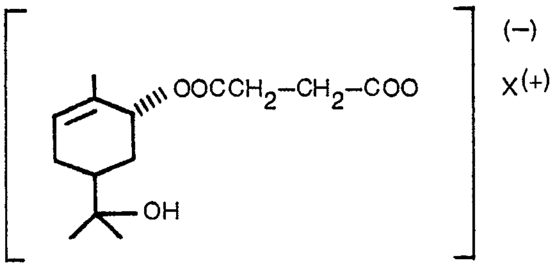

(I)

in which $X^{(+)}$ represents the monovalent cation of erythromycin A, characterized in that a solution of erythromycin A base (III) in an anhydrous chlorinated organic solvent is reacted, at room temperature, with an equimolar amount of butanedioic acid, mono[5-(1-hydroxy-1-methylethyl)-2-methyl-2-cyclohexen-1-yl]ester, then, to the clear solution so obtained, an ether is added in order to precipitate the erythromycin A salt of formula (I) of claim 1, whereupon the solution is concentrated to about one half the volume, cooled and filtered yielding said erythromycin A salt in crystalline form.

2. The process according to claim 1, characterized in that the reaction is carried out at a temperature from 20 to 25°C for 25—35 minutes in anhydrous methylene chloride, using isopropyl ether to precipitate the erythromycin salt.

3. A process for the preparation of the compound of formula (II)

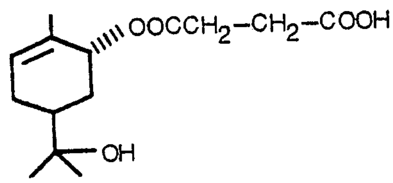

(II)

characterized in that a mixture of d,l-trans-sobrerol in an aprotic solvent is reacted with a small excess of succinic anhydride, in the presence of 4-dimethyl-aminopyridine, stirring the reaction mixture for 8—20 hours, at room temperature, thereafter heating said reaction mixture at the reflux temperature for a 2—5 hours period, then pouring the reaction mixture into a 5% sulphuric acid aqueous solution and stirring it for 50—80 minutes, extracting with ethyl acetate, washing the resulting organic phases with water and drying, the solvent is then concentrated and the reaction mass is cooled, after which it is filtered yielding the compounds of formula (II) in crystalline form.

4. The process according to claim 3, characterized in that the aprotic solvent is tetrahydrofuran and that 4-di-methylaminopyridine is added at an amount of 0.025 mole per mole of d,l-trans-sobrerol, at a temperature of 20—25°C in about 12 hours, and the reaction mixture 3 hours.

5. A process for making a pharmaceutical composition having mucosecretolytic-fluidizing and antibiotic activity, characterized in that the compound of formula I of claim 1 is admixed with one or more pharmacologically acceptable vehicles.

**Patentansprüche für die Vertragsstaaten: BE CH DE FR GB IT LI LU NL SE**

1. Erythromycinsalz des Butandisäure-mono[5-(1-hydroxy-1-methylethyl)-2-methyl-2-cyclohexen-1-yl] esters der Formel:

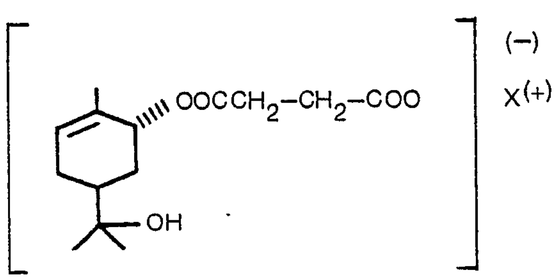

(I)

in welcher $X^{(+)}$ das einwertige Kation des Erythromycins A bedeutet.

2. Verfahren zur Herstellung der Verbindung der Formel (I) nach Anspruch 1, dadurch gekennzeichnet, dass eine Lösung von Erythromycin A Base (III) in einem wasserfreien chlorierten organischen Lösungsmittel mit einer equimolaren Menge an Butandisäure-mono[5-(1-hydroxy-1-methylethyl)-2-methyl-2-cyclohexen-1-yl]ester bei Raumtemperatur umgesetzt wird, wonach zur so erhaltenen klaren Lösung, zur Ausfällung des Erythromycin-A-Salzes der Formel (I) nach Anspruch 1, ein Ether zugegeben wird, wobei die Lösung ungefähr auf die Hälfte seines ursprünglichen Volumens eingeengt, abgekühlt und filtriert wird, wodurch das genannte Erythromycin-A-Salz in Kristallform erhalten wird.

3. Das Verfahren nach Anspruch 2, dadurch gekennzeichnet, dass die Umsetzung bei einer Temperatur von 20 bis 25°C über einem Zeitraum von 25 bis 35 Minuten in wasserfreiem Methylenchlorid durchgeführt wird, wobei Isopropylether zur Ausfällung des Erythromycin-A-Salz verwendet wird.

4. Butandisäure-mono[5-(1-hydroxy-1-methylethyl)-2-methyl-2-cyclohexen-1-yl] ester der Formel:

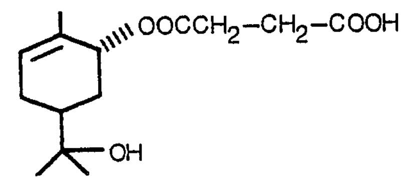

5. Verfahren zur Herstellung der Säure der Formel (II) nach Anspruch 4, dadurch gekennzeichnet, dass ein d,l-trans-Sobrerol-Gemisch, in einem aprotischen Lösungsmittel gelöst, mit einem geringen Überschuss an Bernsteinsäureanhydrid in Gegenwart von 4-dimethyl-aminopyridin bei Raumtemperatur umgesetzt wird, wobei das Reaktionsgemisch während 8 bis 20 Stunden gerführt wird, danach das genannte Reaktionsgemisch bei Rückflusstemperatur über einen Zeitraum von 2 bis 5 Stunden geheizt wird, wonach die Reaktionsmasse in eine 5-prozentige wässrige Schwefelsäurelösung einfliessen lässt und 50 bis 80 Minuten rührt, mit Essigester extrahiert, die erhaltenen organischen Phasen mit Wasser wäscht und trocknet, das Lösungsmittel dann eingeengt und die Reaktionsmasse abgekühlt wird, wonach sie filtriert wird und die Verbindungen der Formel (II) in Kristallform liefert.

6. Das Verfahren nach Anspruch 5, dadurch gekennzeichnet, dass das aprotische Lösungsmittel Tetrahydrofuran ist, und dass 4-dimethyl-aminopyridin in einer Menge von 0,025 Mol pro Mol d,l-trans-Sobrerol bei einer Temperatur von 20 bis 25°C über einem Zeitraum von ungefähr 12 Stunden zugegeben wird, wobei der Reaktionsansatz 3 Stunden unter Rückfluss gekocht wird.

7. Pharmazeutische Zubereitungen mit mucosekretolytisch-verflüssigender und antibiotischer Wirkung, dadurch gekennzeichnet, dass sie als Wirkstoff die Verbindung der Formel (I) nach Anspruch 1 zusammen mit einem oder mehreren pharmakologisch unbedenklichen Träger enthält.

**Patentansprüche für den Vertragsstaat: AT**

1. Verfahren zur Herstellung der Verbindung der Formel (I)

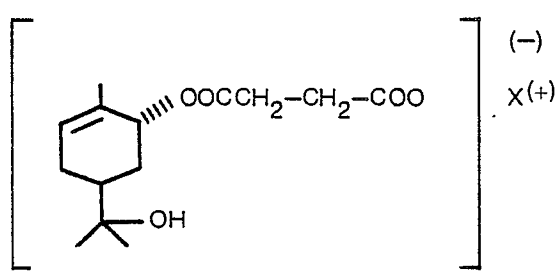

(I)

in welcher $X^{(+)}$ das einwertige Kation des Erythromycins A bedeutet, dadurch gekennzeichnet, dass eine Lösung von Erythromycin A Base (III) in einem wasserfreien chlorierten organischen Lösungsmittel mit einer equimolaren Menge an Butandisäure-mono[5-(1-hydroxy-1-methylethyl)-2-methyl-2-cyclohexen-1-yl]ester bei Raumtemperatur umgesetzt wird, wonach zur so erhaltenen klaren Lösung, zur Ausfällung des Erythromycin-A-Salzes der Formel (I), ein Ether zugegeben wird, wobei die Lösung ungefähr auf die Hälfte seines ursprünglichen Volumens eingeengt, abgekühlt und filtriert wird, wodurch das genannte Erythromycin-A-Salz in Kristallform erhalten wird.

2. Das Verfahren nach Anspruch 1, dadurch gekennzeichnet, dass die Umsetzung bei einer Temperatur von 20 bis 25°C über einem Zeitraum von 25 bis 35 Minuten in wasserfreiem Methylenchlorid durchgeführt wird, wobei Isopropylether zur Ausfallung des Erythromycin-A-Salz verwendet wird.

3. Verfahren zur Herstellung der Verbindung der Formel (II)

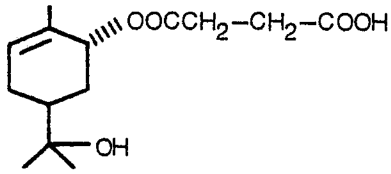

dadurch gekennzeichnet, dass ein d,l-trans-Sobrerol-Gemisch, in einem aprotischen Lösungsmittel gelöst, mit einem geringen Überschuss an Bernsteinsäureanhydrid in Gegenwart von 4-dimethyl-aminopyridin bei Raumtemperatur umgesetzt wird, wobei das Reaktionsgemisch während 8 bis 20 Stunden gerührt wird, danach das genannte Reaktionsgemisch bei Rückflusstemperatur über einen Zeitraum von 2 bis 5 Stunden geheizt wird, wonach die Reaktionsmasse in eine 5-prozentige wässrige Schwefelsäurelösung einfliessen lässt und 50 bis 80 Minuten rührt, mit Essigester extrahiert, die erhaltenen organischen Phasen mit Wasser wäscht und trocknet, das Lösungsmittel dann eingeengt und die Reaktionsmasse abgekühlt wird, wonach sie filtriert wird und die Verbindungen der Formel (II) in Kristallform liefert.

4. Das Verfahren nach Anspruch 3, dadurch gekennzeichnet, dass das aprotische Lösungsmittel Tetrahydrofuran ist, und dass 4-dimethyl-aminopyridin in einer Menge von 0,025 Mol pro Mol d,l-trans-Sobrerol bei einer Temperatur von 20 bis 25°C über einem Zeitraum von ungefähr 12 Stunden zugegeben wird, wobei der Reaktionsansatz 3 Stunden unter Rückfluss gekocht wird.

5. Verfahren zur Herstellung einer pharmazeutischen Zubereitung mit mucosekretolytisch-verflüssigender und antibiotischer Wirkung, dadurch gekennzeichnet, dass sie als Wirkstoff die Verbindung der Formel (I) nach Anspruch 1 zusammen mit einem oder mehreren pharmakologisch unbedenklichen Träger enthält.

**Revendications pour les États contractants: BE CH DE FR GB IT LI LU NL SE**

1. Sel d'érythromycine du mono [5-(1-hydroxy-1-méthyléthyl)-2-méthyl-2-cyclohexène-1-yl]ester de l'acide butanédioique de la formule:

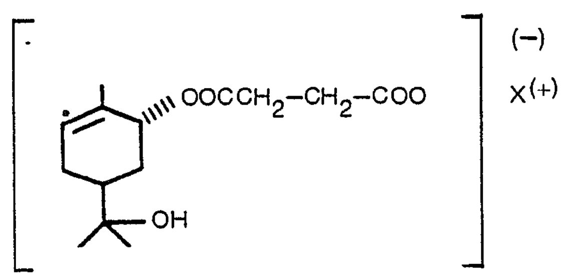

(I)

dans laquelle $X^{(+)}$ représente le cation monovalent de l'érythromycine-A.

2. Procédé pour la préparation du composé de formule (I) de la revendication 1, caractérisé en ce qu'on fait réagir une solution d'érythromycine-A base (III) dans un solvant organique chloruré anhydre, à température ambiante, avec une quantité équimolaire de mono [5-(1-hydroxy-1-méthyléthyl)-2-méthyl-2-cyclohexène-1-yl]ester de l'acide butanédioique, ensuite on ajoute à la solution limpide ainsi obténue un éther afin de précipiter le sel d'érythromycine-A de formule (I) de la revendication 1, après quoi la solution est concentrée jusqu'à environs la moitié de son volume, refroidie et filtrée, donnant ainsi ledit sel d'érythromycine-A en forme cristalline.

3. Procédé selon la revendication 2, caractérisé en ce que la réaction a lieu à une température de 20 à 25°C pendant 25—35 minutes en chlorure de méthylène anhydre, avec l'utilisation d'éther isopropylique pour la précipitation du sel d'érythromycine-A.

4. Mono[5-(1-hydroxy-1-méthyléthyl)-2-méthyl-2-cyclohexène-1-yl]ester de l'acide butanédioique ayant la formule:

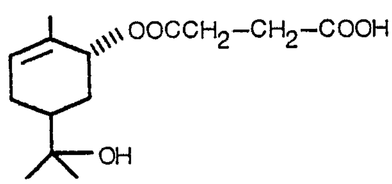

(II)

5. Procédé pour la préparation de l'acide de formule (II) selon la revendication 4, caractérisé en ce qu'on fait réagir un mélange de d,l-trans-sobrerol dans un solvant aprotique avec un petit excès d'anhydride succinique, en présence de 4-diméthyl-aminopyridine, à température ambiante en agitant le mélange de réaction pendant 8—20 heures, en chauffant ensuite ledit mélange de réaction à température de reflux pendant une période de 2—5 heures, en versant ensuite la masse de réaction dans une solution aqueuse à 5% d'acide sulfurique et en l'agitant pendant 50—80 minutes, après quoi on extrait avec de l'acétate d'éthyle, on lave les phases organiques ainsi obténues avec de l'eau et on sèche; le solvant est ensuite concentré et la masse de réaction est refroidie, après quoi elle est filtrée, donnant le composé de formule (II) en forme cristalline.

6. Procédé selon la revendication 5, caractérisé en ce que le solvant aprotique est la tétrahydrofurane et qu'on ajoute 4-diméthyl-aminopyridine dans une quantité de 0,025 mol par mol de d,l-trans-sobrerol, à une température de 20 à 25°C pendant environ 12 heures, en refluant à la masse de réaction pendant 3 heures.

7. Compositions pharmaceutiques ayant une activité mucosécrétolytique-fluidifiante et antibiotique, caractérisé en ce qu'elles contiennent comme ingrédient actif le composé de formule (I) selon la revendication 1, avec un ou plusieurs véhicules pharmacologiquement acceptables.

**Revendications pour l'Etat contractant: AT**

1. Procédé pour la préparation du composé de formule (I)

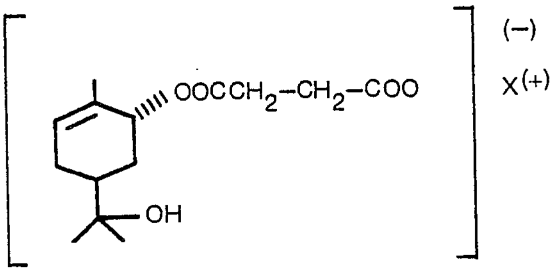

(I)

dans laquelle $X^{(+)}$ représente le cation monovalent de l'érythromycine-A caractérisé en ce qu'on fait réagir une solution d'érythromycine-A base (III) dans un solvant organique chloruré anhydre, à température ambiante, avec une quantité équimolaire de mono [5-(1-hydroxy-1-méthyléthyl)-2-méthyl-2-cyclohexène-1-yl]ester de l'acide butanédioique, ensuite on ajoute à la solution limpide ainsi obténue un éther afin de précipiter le sel d'érythromycine-A de formule (I) de la revendication 1, après quoi la solution est concentrée jusqu'à environ la moitié de son volume, refroidie et filtrée, donnant ainsi ledit sel d'érythromycine-A en forme cristalline.

2. Procédé selon la revendication 1, caractérisé en ce que la réaction a lieu à une température de 20 à 25°C pendant 25—35 minutes en chlorure de méthylène anhydre, avec l'utilisation d'ether isopropylique pour la précipitation du sel d'érythromycine-A.

3. Procédé pour la préparation du composé de formule (II)

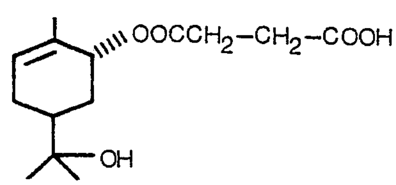

(II)

caractérisé en ce qu'on fait réagir un mélange de d,l-trans-sobrerol dans un solvant aprotique avec un petit excès d'anhydride succinique, en présence de 4-diméthyl-aminopyridine, à température ambiante en agitant le mélange de réaction pendant 8—20 heures, en chauffant ensuite ledit mélange de réaction à température de reflux pendant une période de 2—5 heures, en versant ensuite la masse de réaction dans une solution aqueuse à 5% d'acide sulfurique et en l'agitant pendant 50—80 minutes, après quoi on extrait avec de l'acétate d'éthyle, on lave les phases organiques ainsi obténues avec de l'eau et on sèche; le solvant est ensuite concentré et la masse de réaction est refroidie, après quoi elle est filtrée, donnant le composé de formule (II) en forme cristalline.

4. Procédé selon la revendication 3, caractérisé en ce que le solvant aprotique est la tétrahydrofurane et qu'on ajoute 4-diméthyl-aminopyridine dans une quantité de 0,025 mol par mol de d,l-trans-sobrerol, à une température de 20 à 25°C pendant environ 12 heures, en refluant à la masse de réaction pendant 3 heures.

5. Procédé pour la préparation d'une composition pharmaceutique ayant une activité mucosécrétolytique-fluidifiante et antibiotique, caractérisé en ce que le composé de formule (I) selon la revendication 1 est mélangé avec ou un plusieurs véhicules pharmacologiquement acceptables.